# EUROPÄISCHE PATENTANMELDUNG

(11) **EP 3 563 707 A1**
(43) Veröffentlichungstag der Anmeldung: **06.11.2019**
(21) Anmeldenummer: 19176070.1
(22) Anmeldetag: 02.04.2015
(51) Int. Cl.: A43B 3/00, A43B 7/32, A61B 5/11, A43B 7/36, A43B 23/08

(54) **ARBEITSSCHUH**

(30) Priorität: 30.06.2014 DE 102014212535
(62) Teilanmeldung aus: 15713765.4
(71) Anmelder: Robert Bosch GmbH, 70442 Stuttgart (DE)
(72) Erfinder: Schadow, Joachim, 70563 Stuttgart (DE); Lutz, Manfred, 70794 Filderstadt (DE); Boeck, Cornelius, 73230 Kirchheim (DE); Maute, Joerg, 71069 Sindelfingen (DE); Esenwein, Florian, 70771 Leinfelden-Echterdingen (DE); Barth, Daniel, 70771 Leinfelden-Echterdingen (DE); Stock, Joern, 70711 Leinfelden-Echterdingen (DE)

(57) **Zusammenfassung**

Die Erfindung geht aus von einem Arbeitsschuh, insbesondere von einem Sicherheitsschuh, mit zumindest einer passiven Schutzeinheit (12), die dazu vorgesehen ist, einen Schuhträger passiv zumindest vor mechanischen und/oder elektrischen Belastungen zu schützen, und mit zumindest einer aktiven Schutzeinheit (14), die zumindest eine Sensoreinheit (16) aufweist, die dazu vorgesehen ist, zumindest eine Kenngröße zumindest zu einer Ermöglichung einer Schutzfunktion und/oder einer Komfortfunktion zu erfassen.

Es wird vorgeschlagen, dass die Sensoreinheit (16) zumindest zu einer Erfassung von zumindest einer personenbezogenen Kenngröße und/oder von zumindest einer Umgebungskenngröße vorgesehen ist.

## Beschreibung

### Stand der Technik

Aus KR 2002 0061662 A1 ist bereits ein Arbeitsschuh, insbesondere ein Sicherheitsschuh, bekannt, der eine passive Schutzeinheit, welche dazu vorgesehen ist, einen Schuhträger passiv zumindest vor mechanischen und/oder elektrischen Belastungen zu schützen, und der eine aktive Schutzeinheit umfasst, welche eine Sensoreinheit aufweist, die dazu vorgesehen ist, eine Kenngröße zumindest zu einer Ermöglichung einer Schutzfunktion und/oder einer Komfortfunktion zu erfassen. Hierbei ist die Sensoreinheit dazu vorgesehen, eine Belastungskenngröße eines Zehenschutzkappenelements des Arbeitsschuhs zu einer Ermöglichung einer Überlastungsschutzfunktion zu erfassen.

### Offenbarung der Erfindung

Die Erfindung geht aus von einem Arbeitsschuh, insbesondere von einem Sicherheitsschuh, mit zumindest einer passiven Schutzeinheit, die dazu vorgesehen ist, einen Schuhträger passiv zumindest vor mechanischen und/oder elektrischen Belastungen zu schützen, und mit zumindest einer aktiven Schutzeinheit, die zumindest eine Sensoreinheit aufweist, die dazu vorgesehen ist, zumindest eine Kenngröße zumindest zu einer Ermöglichung einer Schutzfunktion und/oder einer Komfortfunktion zu erfassen.

Es wird vorgeschlagen, dass die Sensoreinheit zumindest zu einer Erfassung von zumindest einer personenbezogenen Kenngröße und/oder von zumindest einer Umgebungskenngröße vorgesehen ist. Unter einer "personenbezogenen Kenngröße" soll hier insbesondere eine Kenngröße verstanden werden, die zumindest einen Vitalwert eines Schuhträgers des Arbeitsschuhs definiert und/oder die abhängig ist von einem Verhalten eines Schuhträgers des Arbeitsschuhs, wie beispielsweise eine Richtung und/oder ein Ort einer Krafteinwirkung eines Schuhträgers auf den Arbeitsschuh. Die personenbezogene Kenngröße kann hierbei als bedienerspezifische Arbeitsschuhbelastungsart, insbesondere einer Sohlenbelastungsart des Arbeitsschuhs, als Trägerbelastung, wie beispielsweise eine Geräuschbelastung und/oder eine Schwingungsbelastung, als Puls eines Schuhträgers des Arbeitsschuhs, als Körpertemperatur eines Schuhträgers des Arbeitsschuhs, als Müdigkeitskenngröße eines Schuhträgers des Arbeitsschuhs, als Trägerausrichtungskenngröße, als Schuhbewegungskenngröße oder als eine andere, einem Fachmann als sinnvoll erscheinende personenbezogene Kenngröße ausgebildet sein. Unter einer "Umgebungskenngröße" soll hier insbesondere eine Kenngröße verstanden werden, die eine den Arbeitsschuh umgebende Umwelt definiert. Hierbei kann die Umgebungskenngröße als Umgebungsdruck, als Umgebungstemperatur, als Umgebungsschallpegel, als Umgebungsgaskenngröße, als Umgebungsspannungskenngröße, als Umgebungsfeuchtigkeitskenngröße, als Umgebungssäure- und/oder Umgebungsbasenkenngröße oder als eine andere, einem Fachmann als sinnvoll erscheinende Umgebungskenngröße ausgebildet sein.

Der Begriff "Sicherheitsschuh" soll hier insbesondere eine Art von Schuh definieren, der zumindest ein Kriterium der in der EN ISO 20345 genannten Kriterien erfüllt. Somit umfasst die passive Schutzeinheit zumindest ein Zehenschutzkappenelement und/oder zumindest ein Durchtrittschutzelement. Das Zehenschutzkappenelement kann hierbei aus Stahl oder aus einem Kunststoff ausgebildet sein. Vorzugsweise weist das Zehenschutzkappenelement insbesondere eine Schutzwirkung gegen eine Stoßenergie von mindestens 200 J auf und eine Schutzwirkung gegen eine Druckbeanspruchung von mindestens 15 kN auf. Das Durchtrittschutzelement kann aus Stahl oder aus einer Gewebestruktur, wie beispielsweise einer Kevlarstruktur, gebildet sein. Vorzugsweise ist das Durchtrittschutzelement als Schicht, insbesondere als Zwischenschicht, einer Sohle des Arbeitsschuhs ausgebildet. Bevorzugt ist die Sohle des Arbeitsschuhs öl- und/oder kraftstoffresistent, elektrisch isolierend und/oder hitzeresistent ausgebildet. Der Arbeitsschuh kann zudem weitere, einem Fachmann als sinnvoll erscheinende Eigenschaften aufweisen, die in der EN ISO 20345 hinsichtlich eines Sicherheitsschuhs genannt sind. Besonders bevorzugt ist die passive Schutzeinheit energieversorgungsfrei ausgebildet. Somit ist die passive Schutzeinheit vorzugsweise dazu vorgesehen, einen Schuhträger des Arbeitsschuhs infolge einer Materialeigenschaft oder einer geometrischen Eigenschaft von Schutzelementen der passiven Schutzeinheit zu schützen. Unter "vorgesehen" soll insbesondere speziell ausgelegt und/oder speziell ausgestattet verstanden werden. Darunter, dass ein Element und/oder eine Einheit zu einer bestimmten Funktion vorgesehen ist, soll insbesondere verstanden werden, dass das Element und/oder die Einheit diese bestimmte Funktion in zumindest einem Anwendungs- und/oder Betriebszustand erfüllen/erfüllt und/oder ausführen/ausführt.

Mittels der erfindungsgemäßen Ausgestaltung des Arbeitsschuhs können vorteilhaft Gefahrensituationen erkannt werden und ein Schuhträger des Arbeitsschuhs kann vorteilhaft vor Gefahren geschützt werden. Zudem kann vorteilhaft ein hoher Tragekomfort erreicht werden, indem zumindest eine Komfortfunktion anhand von Sensordaten überwacht und/oder angepasst wird. Somit kann besonders vorteilhaft ein Arbeitsschuh zur Verfügung gestellt werden, der ein hohes Maß an Trägersicherheit und/oder Trägerkomfort zur Verfügung stellen kann.

Des Weiteren wird vorgeschlagen, dass die Sensoreinheit zumindest ein Beschleunigungssensorelement umfasst, das dazu vorgesehen ist, zumindest eine Beschleunigungskenngröße zu erfassen, die zumindest zu einer Auswertung eines sicheren Stands eines Schuhträgers heranziehbar ist. Das Beschleunigungssensorelement kann hierbei als mehrachsiges Beschleunigungssensorelement, insbesondere als dreiachsiges Beschleunigungssensorelement, als Drehratensensorelement, als piezoelektrisches Beschleunigungssensorelement, als Beschleunigungssensorelement aus einem mikro-elektro-mechanischen System (MEMS) oder als ein anderes, einem Fachmann als sinnvoll erscheinendes Beschleunigungssensorelement ausgebildet sein. Mittels der erfindungsgemäßen Ausgestaltung kann vorteilhaft kostengünstig eine personenbezogene Kenngröße ermittelt werden, die vorzugsweise Rückschlüsse auf einen sicheren Stand eines Schuhträgers zulässt. So ist beispielsweise eine durch ein Muskelzucken eines Schuhträgers hervorgerufene Beschleunigungskenngröße, wie beispielsweise infolge einer Anstrengung einer Muskulatur bei einem Stehen auf Zehenspitzen o. dgl., erfassbar, die zu einer Auswertung eines sicheren Stands eines Schuhträgers heranziehbar ist. Bei einer Anstrengung einer Muskulatur ist beispielsweise ein Muskelzucken in einem Frequenzbereich von 2 bis 12 Hz oder höher möglich, das vorteilhaft zu einer Auswertung über einen sicheren Stand eines Schuhträgers heranziehbar ist. Vorzugsweise ist das Beschleunigungssensorelement hierzu vorteilhaft an einem einem Schuhträger zugewandten Innenbereich des Arbeitsschuhs angeordnet. Zudem kann vorteilhaft eine mittels des Beschleunigungssensorelements erfasste Beschleunigungskenngröße zu einer Auswertung eines Vorliegens eines freien Falls eines Schuhträgers, beispielsweise von einer Leiter, oder zu einer Auswertung eines Ausrutschens eines Schuhträgers auf einem rutschigen Untergrund genutzt werden. Ferner kann vorteilhaft eine mittels des Beschleunigungssensorelements erfasste Beschleunigungskenngröße genutzt werden, um zu erkennen, ob sich ein Schuhträger in einer Gefahrensituation befindet, insbesondere wenn das Beschleunigungssensorelement keine Bewegung erfasst und ein Schuhträger sich über eine bestimmte Zeitspanne in einer Lage, insbesondere in einer zumindest im Wesentlichen horizontalen Lage relativ zu einem Untergrund, nicht verändert.

Vorteilhafterweise umfasst die Sensoreinheit zumindest ein Temperatursensorelement, das dazu vorgesehen ist, zumindest eine Temperaturkenngröße, insbesondere eine Körpertemperatur eines Schuhträgers, eine Untergrundtemperatur und/oder eine Umgebungstemperatur, zu erfassen. Vorzugsweise umfasst die Sensoreinheit somit zumindest ein als Köpertemperatursensorelement ausgebildetes Temperatursensorelement, das dazu vorgesehen ist, eine Körpertemperatur eines Schuhträgers des Arbeitsschuhs zu erfassen. Hierbei ist das als Köpertemperatursensorelement ausgebildete Temperatursensorelement bevorzugt an einem Innenbereich des Arbeitsschuhs angeordnet. Ferner umfasst die Sensoreinheit vorzugsweise zumindest ein als Untergrundtemperatursensorelement ausgebildetes Temperatursensorelement, das dazu vorgesehen ist, eine Untergrundtemperatur eines Untergrunds, auf dem der Arbeitsschuh angeordnet ist und/oder bewegt wird, zu erfassen. Hierbei ist das als Untergrundtemperatursensorelement ausgebildete Temperatursensorelement bevorzugt an der Sohle, insbesondere im Bereich einer Untergrundkontaktfläche der Sohle, angeordnet. Zudem umfasst die Sensoreinheit somit vorzugsweise zumindest ein als Umgebungstemperatursensorelement ausgebildetes Temperatursensorelement, das dazu vorgesehen ist, eine Umgebungstemperatur, insbesondere eine Umgebungslufttemperatur, zu erfassen. Hierbei ist das als Umgebungstemperatursensorelement ausgebildete Temperatursensorelement bevorzugt an einem Außenbereich des Arbeitsschuhs angeordnet. Hierbei kann das Temperatursensorelement direkt in ein Obermaterial des Arbeitsschuhs integriert sein, insbesondere direkt in einen Werkstoff des Obermaterials integriert sein, wie beispielsweise direkt in Fasern eines Obermaterials. Mittels der erfindungsgemäßen Ausgestaltung kann somit vorteilhaft eine Temperaturkenngröße erfasst werden, die zu einer Auswertung hinsichtlich eines körperlichen Zustands eines Schuhträgers des Arbeitsschuhs, hinsichtlich einer Komfortfunktionszuschaltung, wie beispielsweise einer Zuschaltung einer Heizung oder einer Kühlung, und/oder hinsichtlich eines Zustands eines Untergrunds, wie beispielsweise eine Eisbildungsgefahr, genutzt werden. Hierbei ist es denkbar, dass das als Durchtrittschutzelement ausgebildete Schutzelement als Wärmeleitelement ausgebildet ist, an dem beispielsweise ein Heizelement und/oder ein Kühlelement des Arbeitsschuhs angeordnet ist und das als Durchtrittschutzelement ausgebildete Schutzelement infolge einer Ausgestaltung aus einem metallischen Werkstoff eine gute Wärmeleitung ermöglicht. Zudem ist es denkbar, dass bei einer Ausgestaltung des als Durchtrittschutzelement ausgebildeten Schutzelements aus einem leitfähigen Kunststoff beispielsweise durch ein Anlegen einer Spannung das als Durchtrittschutzelement ausgebildete Schutzelement selbst als Heizelement ausgebildet sein kann. Mittels der erfindungsgemäßen Ausgestaltung sind vorteilhaft verschiedenste Temperaturen zu einer Ermöglichung einer hohen Trägersicherheit erfassbar.

Ferner wird vorgeschlagen, dass die Sensoreinheit zumindest ein Drucksensorelement umfasst, das dazu vorgesehen ist, zumindest eine Druckkenngröße, insbesondere eine von einem Schuhträger des Arbeitsschuhs auf eine Sohle wirkende Sohlendruckkenngröße, zu erfassen. Hierbei ist es denkbar, dass die Sensoreinheit lediglich ein einzelnes Drucksensorelement umfasst, das an der Sohle des Arbeitsschuhs angeordnet ist, oder das die Sensoreinheit eine Vielzahl an Drucksensorelementen aufweist, die verteilt an der Sohle angeordnet sind und einen einzelnen Drucksensorbereich oder mehrere Drucksensorbereiche bilden. Besonders bevorzugt weist die Sensoreinheit eine Vielzahl an Drucksensorelementen auf, die rasterartig an der Sohle angeordnet sind. Hierbei ist es denkbar, dass das Drucksensorelement oder die Drucksensorelemente auf einer einer Untergrundkontaktfläche der Sohle abgewandten Seite des Durchtrittschutzelements der passiven Schutzeinheit angeordnet ist/sind. Ferner ist es denkbar, dass das Drucksensorelement oder die Drucksensorelemente zwischen zwei Durchtrittschutzelementen der passiven Schutzeinheit angeordnet ist/sind. Zudem ist es denkbar, dass die rasterartig angeordneten Drucksensorelemente in einem Trägerelement, wie beispielsweise einem Gewebe der aktiven Schutzeinheit angeordnet sind und als Drucksensorelementeinheit in den Arbeitsschuh, insbesondere in einen Fußbettbereich des Arbeitsschuhs, einlegbar sind. Somit ist es denkbar, dass die Drucksensorelementeinheit eine Schuheinlage bildet. Mittels der erfindungsgemäßen Ausgestaltung kann vorteilhaft präzise eine Druckbelastung eines Schuhträgers auf den Arbeitsschuh erfasst werden. Insbesondere infolge einer Verteilung einer Vielzahl an Drucksensorelementen ist vorteilhaft eine präzise Erfassung eines Einwirkungsorts, der zu einer Schutzfunktionsauswertung der erfassten Druckkenngröße nutzbar ist, erreichbar.

Zudem wird vorgeschlagen, dass der Arbeitsschuh zumindest eine Auswerteeinheit umfasst, die zumindest dazu vorgesehen ist, die erfasste Beschleunigungskenngröße zu einer Erkennung eines sicheren Stand eines Schuhträgers auszuwerten und/oder die erfasste Druckkenngröße zu einer Erkennung einer Sohlenbelastungsverteilung auszuwerten. Unter einer "Sohlenbelastungsverteilung" soll hier insbesondere eine Verteilung einer von einem Schuhträger des Arbeitsschuhs auf den Arbeitsschuh, insbesondere auf die Sohle, wirkende Kraft, insbesondere Druckkraft, verstanden werden. Hierbei ist es denkbar, dass die Auswerteeinheit dazu vorgesehen ist, Kenngrößen von einem Paar Arbeitsschuhen auszuwerten oder von einem einzelnen Arbeitsschuh, wobei beispielsweise eine Kommunikation zwischen Auswerteeinheiten der einzelnen Arbeitsschuhe eines Paars Arbeitsschuhen erfolgen kann. Mittels der erfindungsgemäßen Ausgestaltung kann vorteilhaft eine Auswertung hinsichtlich einer Standsicherheit eines Schuhträgers des Arbeitsschuhs erreicht werden. Es kann vorteilhaft eine Auswertung erreicht werden, mittels derer beispielsweise auf ein Knien eines Schuhträgers, auf ein Stehen auf einer Leiter, auf ein vollflächiges Aufliegen der Sohle auf einem Untergrund, auf ein Stehen auf Zehenspitzen usw. geschlossen werden kann.

Des Weiteren wird vorgeschlagen, dass die Sensoreinheit zumindest ein Ortungssensorelement aufweist, das dazu vorgesehen ist, zumindest eine Positionskenngröße, insbesondere zumindest eine globale Positionskenngröße und/oder zumindest eine relative Arbeitsbereichspositionskenngröße, zu erfassen. Die Sensoreinheit umfasst hierzu vorzugsweise zumindest ein als GPS-Sensorelement ausgebildetes Sensorelement, mittels dessen eine globale Position des Arbeitsschuhs erfassbar ist. Es ist jedoch auch denkbar, dass die Sensoreinheit ein anderes, einem Fachmann als sinnvoll erscheinendes Sensorelement zu einer Erfassung einer als globalen Position ausgebildeten Positionskenngröße aufweist, wie beispielsweise ein Kompass-Ortungssensorelement, ein Galileo-Ortungssensorelement, ein GLONASS-Ortungssensorelement, ein Beidou-Ortungssensorelement o. dgl. Zudem weist die Sensoreinheit vorzugsweise zumindest ein als Arbeitsbereichspositionssensorelement ausgebildetes Sensorelement auf, das dazu vorgesehen ist, beispielsweise mittels einer Laufzeitmessung über ein WLAN-Netzwerk oder über ein Mobilfunknetzwerk, eine relative Positionserkennung des Arbeitsschuhs innerhalb eines Arbeitsbereichs zu ermöglichen. Somit kann vorteilhaft eine Erfassung einer Position des Arbeitsschuhs erreicht werden, die beispielsweise bei einem Notfall eine zuverlässige Ortsbestimmung des Arbeitsschuhs und somit eines Schuhträgers des Arbeitsschuhs ermöglichen. Hierbei ist es denkbar, dass eine Erfassung von zumindest einer Positionskenngröße erst erfolgt, wenn eine Gefahrensituation erkannt wird. Somit ist es denkbar, dass eine Erfassung von zumindest einer Positionskenngröße während eines Nichtvorliegens einer Gefahrensituation deaktiviert ist.

Ferner wird vorgeschlagen, dass der Arbeitsschuh zumindest eine Kommunikationseinheit umfasst, die zu einer Kommunikation mit zumindest einer externen Einheit zu einem Austausch von elektronischen Daten vorgesehen ist. Die Kommunikationseinheit ist vorzugsweise als kabellose Kommunikationseinheit ausgebildet. Hierbei kann die Kommunikationseinheit als WLAN-Kommunikationseinheit, als Bluetooth-Kommunikationseinheit, als Funk-Kommunikationseinheit, als RFID-Kommunikationseinheit, als NFC-Einheit, als Infrarot-Kommunikationseinheit, als Mobilfunknetz-Kommunikationseinheit, als Zigbee-Kommunikationseinheit o. dgl. ausgebildet sein. Besonders bevorzugt ist die Kommunikationseinheit zu einer bidirektionalen Datenübertragung vorgesehen. In einer alternativen Ausgestaltung ist die Kommunikationseinheit als kabelgebundene Kommunikationseinheit ausgebildet, wie beispielsweise als LAN-Kommunikationseinheit, als USB-Kommunikationseinheit, als Powerline-Kommunikationseinheit, als Canbus-Kommunikationseinheit, als Ethernet-Kommunikationseinheit, als twisted pair Kabel-Kommunikationseinheit (CAT5 oder CAT6) o. dgl. Es ist jedoch auch denkbar, dass die Kommunikationseinheit alternativ zu einer kabellosen oder zu einer kabelgebundenen Kommunikation zu einer kabellosen und zu einer kabelgebunden Kommunikation mit einer externen Einheit vorgesehen ist. Die externe Einheit kann als Smartphone, als Personal-Computer, als Laptop, als Netbook, als Tablet, als Firmenzentralrechner, als tragbare Werkzeugmaschine, als Ausgabeeinheit, wie beispielsweise als Lautsprecher, als Arbeitskleidung, als Schutzbrille, als Schutzhelm oder als eine andere, einem Fachmann als sinnvoll erscheinende externe Einheit ausgebildet sein. Bei einer Ausgestaltung als Smartphone, als Personal-Computer, als Laptop, als Netbook oder als Tablet ist vorzugsweise eine App zu einer Kommunikation mit der Kommunikationseinheit vorgesehen. Es ist jedoch auch denkbar, dass die externe Einheit als externe, transportable Bedieneinheit, als fest installierte Bedieneinheit an einem Arbeitsplatz eines Bedieners, als fest in einem Raum installierte Synchronisationseinheit eines Einsatzortes, die von einer Zentrale gesteuert werden kann, wie beispielsweise infolge von Firmenvorgaben/Sicherheitsbestimmungen, als Körperkenngrößenüberwachungseinheit oder als weitere, einem Fachmann als sinnvoll erscheinende zentrale oder dezentrale Bedieneinheit, Eingabestation und/oder zentrales oder dezentrales Terminal ausgebildet ist. Es kann somit vorteilhaft eine Synchronisation von elektronischen Daten ermöglicht werden. Wird beispielsweise mittels eines Sensorelements der Sensoreinheit eine Anwesenheit eines Schuhträgers des Arbeitsschuhs, insbesondere ein Tragen des Arbeitsschuhs, erkannt, wird zumindest teilweise automatisch eine Verbindung zwischen der Kommunikationseinheit und der externen Einheit aufgebaut. In der externen Einheit hinterlegte Einstellungen sind somit vorzugsweise direkt auf den Arbeitsschuh und/oder von dem Arbeitsschuh an die externe Einheit übertragbar. Hierbei kann es sich um individuelle Einstellungen eines Schuhträgers oder um Vorgaben einer Firma handeln. Zudem sind/ist mittels der Kommunikationseinheit beispielsweise eine Geräuschbelastung eines Schuhträgers zu einer Kontrolle einer Einhaltung einer Belastungsgrenze und/oder eine eventuelle Bezahlung von Zulagen an eine Firmenzentrale o. dgl. übertragbar. Mittels der erfindungsgemäßen Ausgestaltung kann vorteilhaft eine komfortable, insbesondere zentrale, Einstellung von Kenngrößen erfolgen. Zudem kann vorteilhaft eine Einmannüberwachung ermöglicht werden, so dass eine Firmenzentrale vorteilhaft über einen Zustand eines Schuhträgers des Arbeitsschuhs und insbesondere über etwaige Gefahrensituationen informiert werden kann. Somit kann vorteilhaft ein hohes Maß an Sicherheit für einen Schuhträger des Arbeitsschuhs gewährleistet werden.

Zudem wird vorgeschlagen, dass der Arbeitsschuh zumindest eine Steuer- und/oder Regeleinheit umfasst, die dazu vorgesehen ist, zumindest einen Arbeitsschuhparameter, insbesondere einen Arbeitsschuhkomfortparameter und/oder einen Arbeitsschuhsicherheitsparameter, in Abhängigkeit von der zumindest einen erfassten personenbezogenen Kenngröße und/oder von der zumindest einen erfassten Umgebungskenngröße anzupassen. Hierbei ist es denkbar, dass die Sensoreinheit zumindest ein Sensorelement umfasst, das dazu vorgesehen ist, eine Verschlusskraftkenngröße des Arbeitsschuhs zu erfassen. Zudem könnte die aktive Schutzeinheit zumindest ein Aktorelement umfassen, das infolge einer Datenübertragung zwischen der Steuer- und/oder Regeleinheit und dem Aktorelement dazu vorgesehen ist, die Verschlusskraftkenngröße in Abhängigkeit von der zumindest einen erfassten personenbezogenen Kenngröße und/oder von der zumindest einen erfassten Umgebungskenngröße anzupassen. Ferner ist es auch denkbar, dass die Sensoreinheit zumindest ein Untergrundbeschaffenheitssensorelement umfasst, das dazu vorgesehen ist, eine Untergrundbeschaffenheit zu erfassen. Hierbei könnte die aktive Sensoreinheit zumindest ein Aktorelement umfassen, das infolge einer Datenübertragung zwischen der Steuer- und/oder Regeleinheit und dem Aktorelement dazu vorgesehen ist, eine Sohleneigenschaft, wie beispielsweise weich, fest, mit Stollen, stollenlos usw., der Sohle des Arbeitsschuhs in Abhängigkeit von der zumindest einen erfassten personenbezogenen Kenngröße und/oder von der zumindest einen erfassten Umgebungskenngröße anzupassen. Zudem ist denkbar, dass die Steuer- und/oder Regelung eine aktive Kühlung oder eine aktive Heizung des Arbeitsschuhs in Abhängigkeit von der zumindest einen erfassten personenbezogenen Kenngröße und/oder von der zumindest einen erfassten Umgebungskenngröße anpasst. Weitere, einem Fachmann als sinnvoll erscheinende Anpassungen von Arbeitsschuhkomfortparametern und/oder Arbeitsschuhsicherheitsparametern sind ebenfalls denkbar. Mittels der erfindungsgemäßen Ausgestaltung kann vorteilhaft eine hohe Sicherheit und ein hoher Komfort eines Schuhträgers des Arbeitsschuhs erreicht werden.

Des Weiteren wird vorgeschlagen, dass die Steuer- und/oder Regeleinheit dazu vorgesehen ist, mittels der Kommunikationseinheit auf eine zentrale Datenbank zuzugreifen, in der zumindest eine Sicherheits- und/oder Betriebsbereichsregel hinterlegt sind/ist, die zumindest zu einer Anpassung zumindest eines Arbeitsschuhparameters, insbesondere eines Arbeitsschuhkomfortparameters und/oder eines Arbeitsschuhsicherheitsparameters, und/oder zu einer Ausgabe von Sicherheitsinformationen mittels einer Ausgabeeinheit verwertbar sind/ist. Bevorzugt wertet die Steuer- und/oder Regeleinheit die in der zentralen Datenbank hinterlegten Sicherheits- und/oder Betriebsbereichsregeln automatisch aus und interpretiert die Sicherheits- und/oder Betriebsbereichsregeln automatisch zu einer Anpassung des zumindest einen Arbeitsschuhparameters. Hierbei ist es denkbar, dass der Arbeitsschuh zusätzlich eine Bedieneinheit aufweist, mittels derer eine Anpassung eines Arbeitsschuhparameters von einem Schuhträger des Arbeitsschuhs manuell anpassbar ist, um beispielsweise eine individuelle Einstellung der Sicherheits- und/oder Betriebsbereichsregeln zu ermöglichen. Besonders bevorzugt sind mittels der Kommunikationseinheit zusätzlich zu einem Zugriff auf die zentrale Datenbank mittels der Kommunikationseinheit elektronische Daten mit zumindest einer externen Einheit austauschbar. Somit kann vorzugsweise ein Datenaustausch zwischen dem Arbeitsschuh und weiteren externen Einheiten stattfinden, wie beispielsweise ein Datenaustausch zwischen dem Arbeitsschuh und einer Sensoreinheit einer Arbeitskleidung, wie beispielsweise einem Sicherheitshelm, einem Handschuh, einer Sicherheitsjacke, einer Sicherheitshose usw., einem Smartphone, einem Laptop, einem PC, einem Handheld, einem Tablet, einem Server o. dgl. Hierbei sind bevorzugt insbesondere die mittels der Sensoreinheit der aktiven Schutzeinheit erfassten Kenngrößen und/oder die mittels der Kommunikationseinheit übertragenen Daten austauschbar und/oder zu einer Anpassung des zumindest einen Arbeitsschuhparameters nutzbar. Vorzugsweise ist eine externe Einheit, insbesondere ein Smartphone, als Router ausgebildet, der als Vermittlungsstelle zumindest zwischen der Kommunikationseinheit und der zentralen Datenbank und/oder einer weiteren externen Einheit, vorgesehen ist. Hierbei ist es vorteilhaft ein individuell abgestimmtes Firmensmartphone zu verwenden. Zudem kann infolge einer Verbindung mit einem Netzwerk, wie beispielsweise einem Firmennetzwerk, ein Internetnetzwerk o. dgl., mittels der Steuer- und/oder Regeleinheit über die Kommunikationseinheit geprüft werden, ob für eine als globale Position ausgebildete Positionskenngröße Sicherheitseinstellungen und/oder aktuelle Klimadaten (Wetter) hinterlegt sind. Mittels der erfindungsgemäßen Ausgestaltung kann vorteilhaft eine zumindest teilweise automatische Berücksichtigung von Sicherheits- und/oder Betriebsbereichsregeln zu einer Anpassung des zumindest einen Arbeitsschuhparameters genutzt werden. Somit kann vorteilhaft ein hoher Tragekomfort und eine sichere Einhaltung von Sicherheitsfunktionen gewährleistet werden.

Ferner wird vorgeschlagen, dass die Sensoreinheit zumindest teilweise in oder an einem passiven Schutzelement der passiven Schutzeinheit angeordnet ist. Bevorzugt ist zumindest ein Sensorelement der Sensoreinheit zumindest teilweise im Zehenschutzkappenelement angeordnet. Somit ist zumindest ein passives Schutzelement der passiven Schutzeinheit, in oder an dem die Sensoreinheit angeordnet ist, vorzugsweise als das Zehenschutzkappenelement ausgebildet. Ferner ist zumindest ein Sensorelement der Sensoreinheit zumindest teilweise im Durchtrittschutzelement angeordnet. Somit ist zumindest ein passives Schutzelement der passiven Schutzeinheit, in oder an dem die Sensoreinheit angeordnet ist, vorzugsweise als das Durchtrittschutzelement ausgebildet. Es kann vorteilhaft ein hoher Schutz der Sensoreinheit gegen eine Beschädigung, insbesondere gegen eine Beschädigung von Sensorelementen der Sensoreinheit, erreicht werden.

Zudem wird vorgeschlagen, dass der Arbeitsschuh zumindest eine Energieversorgungseinheit und/oder eine Energiespeichereinheit umfasst, die zumindest zu einer Energieversorgung der aktiven Schutzeinheit vorgesehen sind/ist. Vorzugsweise ist die Energiespeichereinheit als Akkumulatoreinheit ausgebildet. Die Akkumulatoreinheit ist bevorzugt mittels zumindest einer Ladeschnittstelle des Arbeitsschuhs wiederaufladbar. Hierbei kann die Ladeschnittstelle als Induktivladeschnittstelle ausgebildet sein. Es ist jedoch auch denkbar, dass die Ladeschnittstelle als kabelgebundene Ladeschnittstelle ausgebildet ist. Die Energieversorgungseinheit ist vorzugsweise als Umwandlungsenergieversorgungseinheit ausgebildet, die dazu vorgesehen ist, mechanische Energie in elektrische Energie umzuwandeln. Hierbei ist die Energieversorgungseinheit bevorzugt als Piezoenergieversorgungseinheit ausgebildet. Somit ist die Energiespeichereinheit vorzugsweise mittels der Ladeschnittstelle mit der Energieversorgungseinheit verbunden. Mittels der erfindungsgemäßen Ausgestaltung können vorteilhaft autark betreibbare Sicherheitsfunktionen des Arbeitsschuhs realisiert werden.

Des Weiteren wird vorgeschlagen, dass der Arbeitsschuh zumindest eine Ausgabeeinheit zu einer Ausgabe von Informationen zumindest in Abhängigkeit von der zumindest einen erfassten personenbezogenen Kenngröße und/oder von der zumindest einen erfassten Umgebungskenngröße und/oder in Abhängigkeit von mittels einer Kommunikationseinheit übertragenen Informationen umfasst. Die Ausgabeeinheit kann hierbei als haptische, als akustische und/oder als optische Ausgabeeinheit ausgebildet sein. Bevorzugt umfasst die Ausgabeeinheit zumindest ein Ausgabeelement, das am Arbeitsschuh angeordnet ist und zu einer Ausgabe einer Information vorgesehen ist. Die Ausgabeeinheit ist vorzugsweise mittels der Steuer- und/oder Regeleinheit steuerbar und/oder regelbar. Mittels der erfindungsgemäßen Ausgestaltung kann vorteilhaft einem Schuhträger des Arbeitsschuhs eine Information angezeigt werden. Somit kann vorteilhaft ein Schuhträger des Arbeitsschuhs vor einer Gefahrensituation gewarnt werden. Zudem können vorteilhaft andere Personen, die sich im Umfeld des Schuhträgers des Arbeitsschuhs aufhalten ebenfalls vorteilhaft vor einer Gefahrensituation gewarnt werden.

Ferner wird vorgeschlagen, dass der Arbeitsschuh zumindest eine Beleuchtungseinheit zu einer Ausleuchtung eines Arbeitsbereichs umfasst. Besonders bevorzugt ist die Beleuchtungseinheit in einem Zehenbereich des Arbeitsschuhs angeordnet, um einen vor dem Arbeitsschuh befindlichen Arbeitsbereich auszuleuchten. Es ist jedoch auch denkbar, dass die Beleuchtungseinheit alternativ oder zusätzlich in einem Knöchelbereich des Arbeitsschuhs angeordnet ist, um einen vor, neben und/oder hinter dem Arbeitsschuh befindlichen Arbeitsbereich auszuleuchten. Besonders bevorzugt ist die Beleuchtungseinheit als LED-Beleuchtungseinheit ausgebildet. Es ist jedoch auch denkbar, dass die Beleuchtungseinheit eine andere, einem Fachmann als sinnvoll erscheinende Ausgestaltung aufweist, wie beispielsweise eine Ausgestaltung als Edelgas-Beleuchtungseinheit, als Laserlicht-Beleuchtungseinheit o. dgl. Mittels der erfindungsgemäßen Ausgestaltung kann vorteilhaft eine hohe Sicherheit eines Schuhträgers erreicht werden. Es kann vorteilhaft ein sicheres Arbeiten in dunklen oder schlecht ausgeleuchteten Arbeitsbereichen ermöglicht werden. Somit kann vorteilhaft eine gute Sicht auf einen Arbeitsbereich erreicht werden.

Zudem wird vorgeschlagen, dass der Arbeitsschuh zumindest eine Projektionseinheit umfasst, die dazu vorgesehen ist, zumindest eine Information auf einen Untergrund zu projizieren. Besonders bevorzugt ist die Projektionseinheit in einem Zehenbereich des Arbeitsschuhs angeordnet, um eine Information auf einen Untergrund vor dem Arbeitsschuh zu projizieren. Es ist jedoch auch denkbar, dass die Projektionseinheit alternativ oder zusätzlich in einem Knöchelbereich des Arbeitsschuhs angeordnet ist, um eine Information auf einen Untergrund vor, neben und/oder hinter dem Arbeitsschuh zu projizieren. Mittels der erfindungsgemäßen Ausgestaltung kann vorteilhaft eine zuverlässige Ablesbarkeit von Informationen ermöglicht werden. Zudem ist eine große Fläche zu einer Anzeige von Informationen nutzbar.

Des Weiteren wird ein Sicherheitssystem mit zumindest einem erfindungsgemäßen Arbeitsschuh und mit zumindest einer externen Einheit vorgeschlagen, mit der der Arbeitsschuh zu einem Austausch von elektronischen Daten zumindest mittels zumindest einer Kommunikationseinheit des Arbeitsschuhs kommuniziert. Somit kann vorteilhaft eine hohe Sicherheit gewährleistet werden.

Ferner wird vorgeschlagen, dass die zumindest eine externe Einheit als tragbare Werkzeugmaschine ausgebildet ist. Unter einer "tragbaren Werkzeugmaschine" soll hier insbesondere eine Werkzeugmaschine zu einer Bearbeitung von Werkstücken verstanden werden, die von einem Bediener transportmaschinenlos transportiert werden kann. Die tragbare Werkzeugmaschine weist insbesondere eine Masse auf, die kleiner ist als 40 kg, bevorzugt kleiner ist als 10 kg und besonders bevorzugt kleiner ist als 5 kg. Hierbei sind vorzugsweise anhand der zumindest einen personenbezogenen Kenngröße und/oder anhand der zumindest einen Umgebungskenngröße vorzugsweise mittels der Steuer- und/oder Regeleinheit des Arbeitsschuhs oder mittels einer Steuer- und/oder Regeleinheit der tragbaren Werkzeugmaschine Sicherheitsfunktionen der tragbaren Werkzeugmaschine und/oder Sicherheitsfunktionen von an der tragbare Werkzeugmaschine anordenbaren Werkzeugmaschinenzubehöreinheiten, steuerbar und/oder regelbar. Hierbei sind beispielsweise mittels der Steuer- und/oder Regeleinheit des Arbeitsschuhs oder Steuer- und/oder Regeleinheit der tragbaren Werkzeugmaschine Sicherheitsparameter, wie beispielsweise ein Kick-Back-Parameter, ein maximales Drehmoment, eine maximale Drehzahl, eine Schlagenergie, eine Schutzhaubenposition und/oder ein Überrastkupplungsauslösemoment einstellbar. Die Sicherheitsparameter sind hierbei vorzugsweise abhängig von einer Werkzeugmaschinenart. Somit kann voreilhaft eine komfortable Einstellung von Sicherheitsfunktionen ermöglicht werden. Zudem kann vorteilhaft eine hohe Sicherheit eines Schuhträgers bei einer Bedienung einer tragbaren Werkzeugmaschine erreicht werden.

Der erfindungsgemäße Arbeitsschuh und/oder das erfindungsgemäße Sicherheitssystem sollen/soll hierbei nicht auf die oben beschriebene Anwendung und Ausführungsform beschränkt sein. Insbesondere können/kann der erfindungsgemäße Arbeitsschuh und/oder das erfindungsgemäße Sicherheitssystem zu einer Erfüllung einer hierin beschriebenen Funktionsweise eine von einer hierin genannten Anzahl von einzelnen Elementen, Bauteilen und Einheiten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

### Zeichnung

Weitere Vorteile ergeben sich aus der folgenden Zeichnungsbeschreibung. In der Zeichnung ist ein Ausführungsbeispiel der Erfindung dargestellt. Die Zeichnung, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: ein erfindungsgemäßes Sicherheitssystem mit zumindest einem Arbeitsschuh und mit zumindest einer externen Einheit in einer schematischen Darstellung,
- Fig. 2: eine Detailansicht des erfindungsgemäßen Arbeitsschuhs in einer schematischen Darstellung und
- Fig. 3: eine Teilschnittansicht des erfindungsgemäßen Arbeitsschuhs in einer schematischen Darstellung.

### Beschreibung des Ausführungsbeispiels

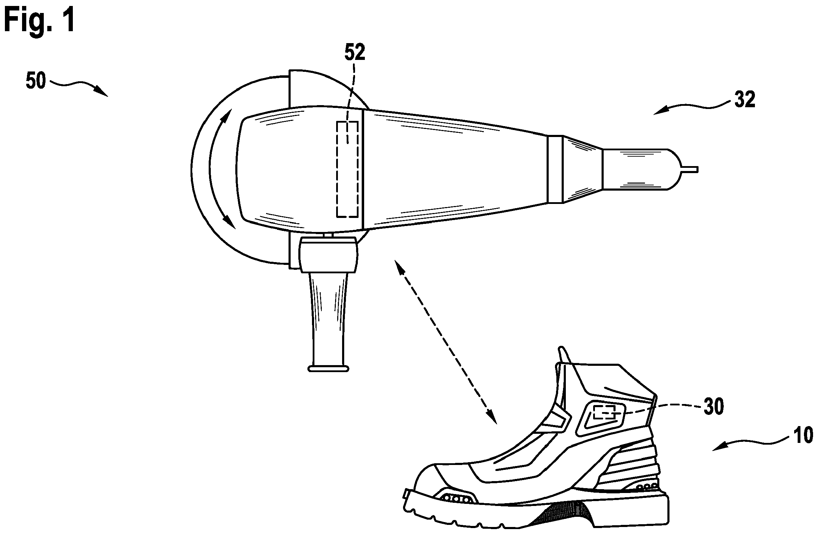

Figur 1 zeigt ein Sicherheitssystem 50 mit zumindest einem Arbeitsschuh 10 und mit zumindest einer externen Einheit 32, mit der der Arbeitsschuh 10 zu einem Austausch von elektronischen Daten zumindest mittels zumindest einer Kommunikationseinheit 30 des Arbeitsschuhs 10 kommuniziert. Insgesamt weist das Sicherheitssystem 50 zumindest zwei Arbeitsschuhe 10 auf, die zusammen ein Paar bilden (in Figur 1 lediglich ein Arbeitsschuh 10 dargestellt). Es ist jedoch auch denkbar, dass lediglich ein Arbeitsschuh 10 eines Arbeitsschuhpaars mit einer Kommunikationseinheit 30 und/oder weiteren Einheiten ausgebildet ist, um Kenngrößen zu erfassen und/oder elektronische Daten mit der externen Einheit 32 auszutauschen. Die externe Einheit 32 ist als tragbare Werkzeugmaschine ausgebildet. Es ist jedoch auch denkbar, dass die externe Einheit 32 eine andere, einem Fachmann als sinnvoll erscheinende Ausgestaltung aufweist.

Die als tragbare Werkzeugmaschine ausgebildete externe Einheit 32 ist in Figur 1 beispielhaft als Winkelschleifmaschine ausgebildet. Es ist jedoch auch denkbar, dass die als tragbare Werkzeugmaschine ausgebildete externe Einheit 32 als eine andere, einem Fachmann als sinnvoll erscheinende Werkzeugmaschine ausgebildet ist, wie beispielsweise als Kreissäge, als Bohrmaschine, als Schlagbohrmaschine, als Bohr- und/oder Meißelhammer o. dgl. Zu einer Kommunikation mit der Kommunikationseinheit 30 des Arbeitsschuhs 10 umfasst die externe Einheit 32 zumindest eine Gegenkommunikationseinheit 52, die zumindest hinsichtlich einer Datenübertragungsmethode korrespondierend zur Kommunikationseinheit 30 des Arbeitsschuhs 10 ausgebildet ist. Ferner kann die externe Einheit 32 zumindest eine Sensoreinheit aufweisen, die zur Erfassung von zumindest einer Kenngröße vorgesehen ist, wobei die erfasste Kenngröße mittels eines Zusammenwirkens der Gegenkommunikationseinheit 52 und der Kommunikationseinheit 30 an den Arbeitsschuh 10 zu einer Weiterverarbeitung und/oder zu einer Speicherung übertragbar ist.

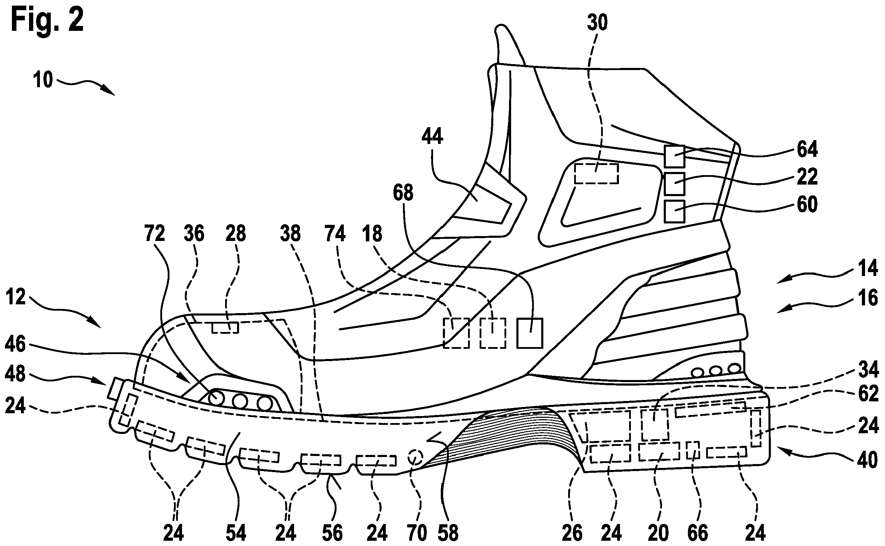

Figur 2 zeigt eine Detailansicht des Arbeitsschuhs 10. Der Arbeitsschuh 10 ist hierbei als Sicherheitsschuh ausgebildet. Der Arbeitsschuh 10 umfasst somit zumindest eine passive Schutzeinheit 12, die dazu vorgesehen ist, einen Schuhträger (hier nicht näher dargestellt) passiv zumindest vor mechanischen und/oder elektrischen Belastungen zu schützen. Hierbei umfasst die passive Schutzeinheit 12 zumindest ein als Zehenschutzkappenelement ausgebildetes Schutzelement 36. Das als Zehenschutzkappenelement ausgebildete Schutzelement 36 ist, auf eine, einem Fachmann bereits bekannte Art und Weise in einem Zehenbereich des Arbeitsschuhs 10 angeordnet. Hierbei ist das als Zehenschutzkappenelement ausgebildete Schutzelement 36 vorzugweise aus einem metallischen Werkstoff ausgebildet. Es ist jedoch auch denkbar, dass das als Zehenschutzkappenelement ausgebildete Schutzelement 36 aus einem Kunststoff ausgebildet ist. Ferner umfasst die passive Schutzeinheit 12 zumindest ein als Durchtrittschutzelement ausgebildetes Schutzelement 38. Das als Durchtrittschutzelement ausgebildete Schutzelement 38 ist, auf eine, einem Fachmann bereits bekannte Art und Weise in oder an einer Sohle 54 des Arbeitsschuhs 10 angeordnet. Hierbei ist das als Durchtrittschutzelement ausgebildete Schutzelement 38 vorzugsweise aus einem metallischen Werkstoff ausgebildet. Es ist jedoch auch denkbar, dass das als Durchtrittschutzelement ausgebildete Schutzelement 38 aus einem Kunststoff oder aus einer Gewebestruktur, wie beispielsweise aus einer Kevlarstruktur, ausgebildet ist.

Ferner umfasst der Arbeitsschuh 10 zumindest eine aktive Schutzeinheit 14, die zumindest eine Sensoreinheit 16 aufweist, die dazu vorgesehen ist, zumindest eine Kenngröße zumindest zu einer Ermöglichung einer Schutzfunktion und/oder einer Komfortfunktion zu erfassen. Die Sensoreinheit 16 ist zumindest zu einer Erfassung von zumindest einer personenbezogenen Kenngröße und/oder von zumindest einer Umgebungskenngröße vorgesehen. Hierbei umfasst die Sensoreinheit 16 zumindest ein Temperatursensorelement 18, das dazu vorgesehen ist, zumindest eine Temperaturkenngröße zu erfassen. Das Temperatursensorelement 18 ist als Körpertemperatursensorelement ausgebildet, das dazu vorgesehen ist, eine Körpertemperatur eines Schuhträgers zu erfassen. Das Temperatursensorelement 18 ist hierzu an einem Innenbereich, insbesondere einem Schuhträger zugewandten und bei einem Tragen des Arbeitsschuhs 10 direkt an einem Schuhträger anliegenden Innenbereich, des Arbeitsschuhs 10 angeordnet. Hierbei kann das als Körpertemperatursensorelement ausgebildete Temperatursensorelement 18 als Kontaktsensorelement ausgebildet sein, das infolge eines direkten Kontakts eine Temperaturkenngröße erfasst, oder das als Körpertemperatursensorelement ausgebildete Temperatursensorelement 18 kann als kontaktloses Sensorelement ausgebildet sein, das eine Temperaturkenngröße mittels elektromagnetischer Wellen, wie beispielsweise Infrarotwellen, erfasst.

Des Weiteren umfasst die Sensoreinheit 16 zumindest ein weiteres Temperatursensorelement 20, das dazu vorgesehen ist, zumindest eine weitere Temperaturkenngröße zu erfassen. Das weitere Temperatursensorelement 20 ist hierbei als Untergrundtemperatursensorelement ausgebildet, das dazu vorgesehen ist, eine Untergrundtemperatur eines Untergrunds (hier nicht näher dargestellt) zu erfassen, auf dem der Arbeitsschuh 10 angeordnet ist. Hierzu ist das weitere Temperatursensorelement 20 an der Sohle 54 des Arbeitsschuhs 10 angeordnet. Das weitere Temperatursensorelement 20 kann als Kontaktsensorelement oder als kontaktloses Sensorelement ausgebildet sein. Hierbei kann das weitere Temperatursensorelement 20 an einer einem Untergrund zugewandten Sohlenfläche 56 der Sohle 54 angeordnet sein oder das weitere Temperatursensorelement 20 kann an einer quer zur Sohlenfläche 56 verlaufenden Seitenfläche 58 der Sohle 54 angeordnet sein, wobei das weitere Temperatursensorelement 20 einen in Richtung eines Untergrunds ausgerichteten Erfassungswinkel aufweist.

Zudem umfasst die Sensoreinheit 16 zumindest ein zusätzliches Temperatursensorelement 22, das dazu vorgesehen ist, zumindest eine zusätzliche Temperaturkenngröße zu erfassen. Das zusätzliche Temperatursensorelement 22 ist hierbei als Umgebungstemperatursensorelement ausgebildet, das dazu vorgesehen ist, eine Umgebungstemperatur, insbesondere eine Umgebungslufttemperatur, einer den Arbeitsschuh 10 umgebenden Umwelt zu erfassen. Hierzu ist das zusätzliche Temperatursensorelement 22 an einer Außenseite des Arbeitsschuhs 10 angeordnet. Das zusätzliche Temperatursensorelement 22 kann hierbei direkt in einen Werkstoff eines Obermaterials des Arbeitsschuhs 10 integriert sein. Des Weiteren umfasst die Sensoreinheit 16 zumindest ein Elektrosensorelement 66, das dazu vorgesehen ist, zumindest eine elektrische Kenngröße zu erfassen. Das Elektrosensorelement 66 ist als kapazitives Sensorelement ausgebildet. Hierbei ist das Elektrosensorelement 66 dazu vorgesehen, eine als Widerstand ausgebildete elektrische Kenngröße zu erfassen. Das Elektrosensorelement 66 ist hierzu an der Sohle 54 des Arbeitsschuhs 10 angeordnet. Somit ist das Elektrosensorelement 66 dazu vorgesehen, eine Leitfähigkeit eines Untergrunds zu erfassen. Es ist jedoch auch denkbar, dass das Elektrosensorelement 66 eine andere, einem Fachmann als sinnvoll erscheinende Ausgestaltung aufweist oder dass das Elektrosensorelement 66 an einer anderen, einem Fachmann als sinnvoll erscheinenden Position am Arbeitsschuh 10 angeordnet ist. Zudem ist es denkbar, dass die Sensoreinheit 16 eine von einem einzelnen Elektrosensorelement 66 abweichende Anzahl an Elektrosensorelementen 66 aufweist. Ferner umfasst die Sensoreinheit 16 zumindest ein weiteres Elektrosensorelement 70, das dazu vorgesehen ist, zumindest eine weitere elektrische Kenngröße zu erfassen. Hierbei ist das Elektrosensorelement 70 dazu vorgesehen, eine als Spannung ausgebildete elektrische Kenngröße zu erfassen.

Die Sensoreinheit 16 umfasst zudem zumindest ein Gassensorelement 68, das dazu vorgesehen ist, zumindest eine Umgebungsgaskenngröße zu erfassen. Das Gassensorelement 68 ist an der Außenseite des Arbeitsschuhs 10 angeordnet. Somit ist vorteilhaft infolge einer Erfassung zumindest einer Umgebungsgaskenngröße eine Gaszusammensetzung einer Umgebungsluft auswertbar. Ein Schuhträger kann somit vorteilhaft vor einer gefährlichen Gaszusammensetzung einer Umgebungsluft, wie beispielsweise eine Gaszusammensetzung der Umgebungsluft mit einem hohen Kohlendioxid- oder Kohlenmonoxidanteil, gewarnt werden oder es ist eine Gaszusammensetzung ausgebbar, insbesondere mittels einer Ausgabeeinheit 44 des Arbeitsschuhs 10.

Die Sensoreinheit 16 umfasst ferner zumindest ein Drucksensorelement 24, das dazu vorgesehen ist, zumindest eine Druckkenngröße, insbesondere eine von einem Schuhträger auf die Sohle 54 des Arbeitsschuhs 10 wirkende Sohlendruckkenngröße, zu erfassen. Vorzugsweise umfasst die Sensoreinheit 16 eine Vielzahl an Drucksensorelementen 24, die an der Sohle 54 des Arbeitsschuhs 10 angeordnet sind. Die Drucksensorelemente 24 sind bereichsweise verteilt an der Sohle 54 des Arbeitsschuhs 10 angeordnet. Hierbei sind einige der Drucksensorelemente 24 in einem Vorderfußbereich der Sohle 54 des Arbeitsschuhs 10 angeordnet. Einige der Drucksensorelemente 24 sind in einem Mittelfußbereich der Sohle 54 des Arbeitsschuhs 10 angeordnet. Zudem sind einige der Drucksensorelemente 24 in einem Fersenbereich der Sohle 54 des Arbeitsschuhs 10 angeordnet. Vorzugsweise sind die in dem Vorderfußbereich, in dem Mittelfußbereich und in dem Fersenbereich der Sohle 54 des Arbeitsschuhs 10 angeordnete Drucksensorelemente 24 unabhängig voneinander auswertbar. Es ist jedoch auch denkbar, das die Drucksensorelemente 24 gleichmäßig verteilt über einen Gesamtbereich der Sohle 54 des Arbeitsschuhs 10 angeordnet sind. Hierbei ist es denkbar, dass die Drucksensorelemente 24 auf einer der Sohlenfläche 56 abgewandten Seite des als Durchtrittschutzelement ausgebildeten Schutzelements 38 der passiven Schutzeinheit 12 an der Sohle 54 des Arbeitsschuhs 10 angeordnet oder dass die Drucksensorelemente 24 in die Sohle 54 des Arbeitsschuhs 10 integriert sind. Ferner ist es denkbar, dass die Drucksensorelemente 24 eine aus dem Arbeitsschuh 10 herausnehmbare Schuheinlage bilden, die mittels einer kabellosen oder einer kabelgebundenen Verbindung an den Arbeitsschuh 10 anschließbar sind.

Der Arbeitsschuh 10 umfasst des Weiteren zumindest eine Auswerteeinheit 26, die zumindest dazu vorgesehen ist, die erfasste Druckkenngröße zu einer Erkennung einer Sohlenbelastungsverteilung auszuwerten. Hierbei ist die Auswerteeinheit 26 insbesondere dazu vorgesehen, alle mittels der Sensoreinheit 16 erfassten Kenngrößen zu einer Weiterverarbeitung auszuwerten. Zudem kann die Auswerteeinheit 26 dazu vorgesehen sein, Druckkenngrößen von beiden Arbeitsschuhen 10 des Arbeitsschuhpaars auszuwerten oder lediglich die Drucckenngröße eines einzelnen Arbeitsschuhs 10 des Arbeitsschuhpaars auszuwerten. Hierbei ist infolge einer Auswertung der erfassten Druckkenngröße, insbesondere einer von einem Schuhträger auf die Sohle 54 des Arbeitsschuhs 10 wirkende Sohlendruckkenngröße, auf ein Knien eines Schuhträgers, auf ein Stehen auf einer Leiter, auf ein vollflächiges Aufliegen der Sohle 54 auf einem Untergrund, auf ein Stehen auf Zehenspitzen usw. schließbar. Somit kann vorteilhaft mittels einer Auswertung der erfassten Druckkenngröße, insbesondere einer von einem Schuhträger auf die Sohle 54 des Arbeitsschuhs 10 wirkenden Sohlendruckkenngröße, auf eine Ausrichtung des Arbeitsschuhs 10 und/oder eine Belastungsart des Arbeitsschuhs 10 infolge einer Haltung eines Schuhträgers geschlossen werden.

Ferner umfasst die Sensoreinheit 16 zumindest ein weiteres Drucksensorelement 60, das dazu vorgesehen ist, eine als Umgebungsdruckkenngröße ausgebildete Druckkenngröße zu erfassen. Somit ist beispielsweise mittels der Auswerteeinheit 26 vorteilhaft auf eine Höhe schließbar, in der sich der Arbeitsschuh 10 befindet. Das weitere Drucksensorelement 60 ist hierbei an der Außenseite des Arbeitsschuhs 10 angeordnet. Die Sensoreinheit 16 kann zudem weitere, einem Fachmann als sinnvoll erscheinende Drucksensorelemente 24 umfassen, die zu einer Erfassung von zumindest einer Druckkenngröße vorgesehen sind.

Ferner umfasst die Sensoreinheit 16 zumindest ein Ortungssensorelement 28, das dazu vorgesehen ist, zumindest eine Positionskenngröße, insbesondere zumindest eine globale Positionskenngröße und/oder zumindest eine relative Arbeitsbereichspositionskenngröße, zu erfassen. Hierzu umfasst die Sensoreinheit 16 zumindest ein als Globalortungssensorelement ausgebildetes Ortungssensorelement 28, das dazu vorgesehen ist, eine globale Positionskenngröße zu erfassen. Das als Globalortungssensorelement ausgebildete Ortungssensorelement 28 ist vorzugsweise als GPS-Ortungselement ausgebildet. Zudem umfasst die Sensoreinheit 16 zumindest ein Arbeitsbereichsortungssensorelement 64, das dazu vorgesehen ist, zumindest eine relative Arbeitsbereichspositionskenngröße zu erfassen. Das Arbeitsbereichsortungssensorelement 64 ist vorzugsweise dazu vorgesehen, zumindest eine Ausrichtung, wie beispielsweise eine horizontale oder eine vertikale Ausrichtung, des Arbeitsschuhs 10 zu erfassen. Das Arbeitsbereichsortungssensorelement 64 ist somit zumindest als Lagesensorelement, wie beispielsweise als mehrachsiges Beschleunigungssensorelement, als Gyrosensorelement, als Drehratensensorelement usw., ausgebildet. Es ist jedoch auch denkbar, dass die Sensoreinheit 16 zusätzlich oder alternativ zu einer Erfassung einer Arbeitsbereichspositionskenngröße mittels des Arbeitsbereichsortungssensorelements 64 zumindest eine relative Arbeitsbereichspositionskenngröße mittels einer Laufzeitmessung von Signalen der Kommunikationseinheit 30 erfasst. Somit ist beispielsweise infolge einer Auswertung der Positionskenngröße mittels der Auswerteeinheit 26 erkennbar, in welcher räumlichen Lage sich ein Schuhträger befindet. Hierdurch kann vorteilhaft darauf geschlossen werden, ob sich ein Schuhträger in einer Gefahrensituation befindet, insbesondere wenn ein Beschleunigungssensorelement 74 der Sensoreinheit 16 keine Bewegung erfasst und ein Schuhträger sich über eine bestimmte Zeitspanne in einer Lage nicht verändert. Ferner ist mittels des Beschleunigungssensorelements 74 beispielsweise eine als Muskelzucken ausgebildete Beschleunigungskenngröße erfassbar, die zu einer Erkennung einer Gefahrensituation eines Schuhträgers heranziehbar ist, wie beispielsweise eine Erkennung eines Schwächeanfalls, eine Erkennung eines sicheren Stands, wie beispielsweise eines sicheren Stands auf einer Leiter, auf Zehenspitzen usw., o. dgl. Somit umfasst die Sensoreinheit 16 zumindest das Beschleunigungssensorelement 74, das dazu vorgesehen ist, zumindest eine Beschleunigungskenngröße zu erfassen, die zumindest zu einer Auswertung eines sicheren Stands eines Schuhträgers heranziehbar ist. Hierbei ist die erfasste Beschleunigungskenngröße zumindest zu einer Erkennung eines sicheren Stands eines Schuhträgers mittels der Auswerteeinheit 26 auszuwerten. Zudem kann die Sensoreinheit 16 weitere, einem Fachmann als sinnvoll erscheinende Sensorelemente umfassen, wie beispielsweise ein Feuchtigkeitssensorelement, ein pH-Wert-Sensorelement, ein Pulssensorelement, ein Blutdrucksensorelement usw., die dazu vorgesehen sind, zumindest eine personenbezogene Kenngröße und/oder zumindest eine Umgebungskenngröße zu erfassen, die mittels der Auswerteeinheit 26 auswertbar ist.

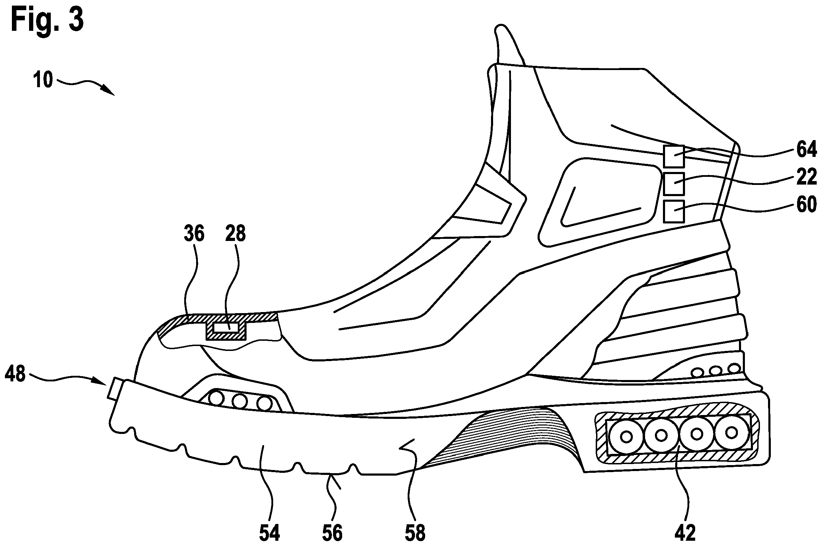

Die Sensoreinheit 16 ist vorzugsweise zumindest teilweise in oder an einem der passiven Schutzelemente 36, 38 der passiven Schutzeinheit 12 angeordnet. Hierbei ist es denkbar, dass zumindest ein Sensorelement, wie beispielsweise das als Globalortungssensorelement ausgebildete Ortungssensorelement 28, der Sensoreinheit 16 in dem als Zehenschutzkappenelement ausgebildeten Schutzelement 36 angeordnet ist (Figur 3). Somit kann das als Zehenschutzkappenelement ausgebildete Schutzelement 36 ein Gehäuse der Sensoreinheit 16 bilden. Es ist zudem denkbar, dass weitere Einheiten des Arbeitsschuhs 10 zumindest teilweise in oder an der passiven Schutzeinheit 12 angeordnet sind, wie beispielsweise eine Energieversorgungseinheit 40 des Arbeitsschuhs 10, eine Energiespeichereinheit 42 des Arbeitsschuhs 10 usw. Zudem ist es denkbar, dass die Sensoreinheit 16 in einer alternativen Ausgestaltung zumindest teilweise an dem als Durchtrittschutzelement ausgebildeten Schutzelement 38 angeordnet ist, wobei das als Durchtrittschutzelement ausgebildete Schutzelement 38 bei einer Ausgestaltung aus einem metallischen Werkstoff als Kühlkörper für die Sensoreinheit 16 vorgesehen sein kann.

Der Arbeitsschuh 10 umfasst zumindest die Kommunikationseinheit 30, die zu einer Kommunikation mit zumindest der externen Einheit 32 zu einem Austausch von elektronischen Daten vorgesehen ist. Hierbei ist es denkbar, dass die elektronischen Daten als Rohdaten der Sensoreinheit 16 ausgebildet sind oder dass die elektronischen Daten bereits mittels der Auswerteeinheit 26 ausgewertete Daten sind. Die Kommunikationseinheit 30 ist dazu vorgesehen, während eines Tragens des Arbeitsschuhs 10 automatisch zu prüfen, ob eine geeignete externe Einheit 32 zu einer Kommunikation in Reichweite ist. Nach einer Erkennung und einer Verbindung mit einer geeigneten externen Einheit 32 beginnt ein Austausch von elektronischen Daten. Bei der in Figur 1 dargestellten externen Einheit 32, die als tragbare Werkzeugmaschine, insbesondere als Winkelschleifmaschine, ausgebildet ist, ist es denkbar, dass infolge einer Auswertung von zumindest einer mittels der Sensoreinheit 16 erfassten personenbezogenen Kenngröße und/oder Umgebungskenngröße mittels der Auswerteeinheit 26 ein Arbeiten auf einer Leiter erkannt wird und dass infolge einer Kommunikation zwischen der als tragbare Werkzeugmaschine ausgebildeten externen Einheit 32 dem Arbeitsschuh 10 eine Kickback-Einstellung der als tragbare Werkzeugmaschine ausgebildeten externen Einheit 32 zumindest teilweise automatisch anpassbar ist. Somit kann vorteilhaft eine Verletzungsgefahr für einen Schuhträger bei einem plötzlichen Verklemmen eines Einsatzwerkzeugs gering gehalten werden und es kann somit vorteilhaft ein Sturz von der Leiter verhindert werden.

Des Weiteren ist beispielsweise infolge einer Auswertung von zumindest einer mittels der Sensoreinheit 16 erfassten personenbezogenen Kenngröße und/oder Umgebungskenngröße mittels der Auswerteeinheit 26 ein freier Fall eines Schuhträgers von einer Leiter erkennbar. Hierzu ist eine mittels des Beschleunigungssensorelements 74 der Sensoreinheit 16 erfasste Beschleunigungskenngröße von der Auswerteeinheit 26 auswertbar. Mittels der Kommunikationseinheit 30 ist daraufhin ein Erkennen des freien Falls an externe Einheiten 32 weiterleitbar, wobei beispielsweise eine als tragbare Werkzeugmaschine ausgebildete externe Einheit 32 automatisch abschaltbar ist, um eine Gefährdung eines Schuhträgers zu reduzieren und mögliche Verletzungen zu vermeiden. Ferner ist beispielsweise infolge einer Auswertung von zumindest einer mittels der Sensoreinheit 16 erfassten personenbezogenen Kenngröße und/oder Umgebungskenngröße mittels der Auswerteeinheit 26 ein Aufprall eines Schuhträgers nach einem Sturz erkennbar. Hierzu sind erfasste Druckkenngrößen und Lagekenngrößen der Sensoreinheit 16 auswertbar. Es kann somit vorteilhaft erkannt werden, ob ein Schuhträger noch steht, liegt oder ob er sich beim Sturz verletzt haben könnte. Zudem kann eine Präzisierung erfolgen, ob beispielsweise eine Ohnmacht vorliegt. Hierzu sind erfasste Vitalkennwerte, wie beispielsweise eine Pulskenngröße, der Sensoreinheit 16 auswertbar. Eine Unfall- und/oder Gefahrenmeldung kann mittels der Kommunikationseinheit 30 an eine als Leitzentrale ausgebildete externe Einheit (hier nicht näher dargestellt) erfolgen oder an eine als Unfallmeldestelle externe Einheit (hier nicht näher dargestellt) abgesetzt werden. Ist eine Kommunikation mittels der Kommunikationseinheit 30 deaktiviert und/oder unmöglich, sind die elektronischen Daten in einer Speichereinheit (hier nicht näher dargestellt) des Arbeitsschuhs 10 ablegbar. Somit kann die Speichereinheit vorteilhaft als Datarecorder/Unfallschreiber genutzt werden, der z.B. die Daten einer definierten Zeiteinheit speichert und diese fortwährend überschreibt.

Des Weiteren umfasst der Arbeitsschuh 10 zumindest eine Steuer- und/oder Regeleinheit 34, die dazu vorgesehen ist, zumindest einen Arbeitsschuhparameter, insbesondere einen Arbeitsschuhkomfortparameter und/oder einen Arbeitsschuhsicherheitsparameter, in Abhängigkeit von der zumindest einen erfassten personenbezogenen Kenngröße und/oder von der zumindest einen erfassten Umgebungskenngröße anzupassen. Hierbei ist es denkbar, dass mittels der Steuer- und/oder Regeleinheit 34 beispielsweise eine Heizfunktion einer Heizeinheit (hier nicht näher dargestellt) oder eine Kühlfunktion einer Kühleinheit (hier nicht näher dargestellt) des Arbeitsschuhs 10 in Abhängigkeit von der zumindest einen erfassten personenbezogenen Kenngröße und/oder von der zumindest einen erfassten Umgebungskenngröße anpassbar ist. Ferner ist es auch denkbar, dass infolge eines Austauschs von elektronischen Daten mittels der Kommunikationseinheit 30 eine Anpassung zumindest eines Arbeitsschuhparameters, insbesondere eines Arbeitsschuhkomfortparameters und/oder eines Arbeitsschuhsicherheitsparameters, durch die Steuer- und/oder Regeleinheit 34 erfolgt. Hierbei könnte beispielsweise eine Heizfunktion einer Heizeinheit (hier nicht näher dargestellt) oder eine Kühlfunktion einer Kühleinheit (hier nicht näher dargestellt) des Arbeitsschuhs 10 mittels einer Kommunikation mit einem Smartphone steuer- und/oder regelbar sein.

Ferner ist die Steuer- und/oder Regeleinheit 34 dazu vorgesehen, mittels der Kommunikationseinheit 30 auf eine zentrale Datenbank zuzugreifen, in der zumindest eine Sicherheits- und/oder Betriebsbereichsregel hinterlegt sind/ist, die zumindest zu einer Anpassung zumindest eines Arbeitsschuhparameters und/oder zu einer Ausgabe von Sicherheitsinformationen mittels der Ausgabeeinheit 44 des Arbeitsschuhs 10 verwertbar sind/ist. Somit ist beispielsweise ein Schuhträger mittels der Ausgabeeinheit 44 darüber informierbar, welche Sicherheits- und/oder Betriebsbereichsregel in einem Bereich, in dem er sich befindet, gelten und einhaltbar sind.

Des Weiteren umfasst der Arbeitsschuh 10 zu einer Energieversorgung der aktiven Schutzeinheit 14 zumindest die Energieversorgungseinheit 40 und/oder die Energiespeichereinheit 42 (Figuren 2 und 3). Die Energieversorgungseinheit 40 ist hierbei als Piezoenergieversorgungseinheit ausgebildet, die zumindest ein Piezoelement 62 aufweist. Das Piezoelement 62 ist hierbei an der Sohle 54 des Arbeitsschuhs 10 angeordnet. In einer hier nicht näher dargestellten Ausgestaltung des Arbeitsschuhs 10 ist das als Durchtrittschutzelement ausgebildete Schutzelement 38 zweilagig ausgebildet, wobei das Piezoelement 62 zwischen zwei Lagen des als Durchtrittschutzelement ausgebildeten Schutzelements 38 angeordnet ist. Hierbei umfasst die Energieversorgungseinheit 40 vorzugsweise eine Vielzahl an Piezoelementen 62, die zwischen den zwei Lagen des als Durchtrittschutzelement ausgebildeten Schutzelements 38 verteilt angeordnet sind. Die Energieversorgungseinheit 40 kann jedoch auch alternativ als Induktivenergieversorgungseinheit oder als Kabelenergieversorgungseinheit ausgebildet sein. Zudem ist die Energieversorgungseinheit 40 auf eine, einem Fachmann bereits bekannte Art und Weise mittels einer Energieversorgungsleitung (hier nicht näher dargestellt) mit der Energiespeichereinheit 42 verbunden. Die Energiespeichereinheit 42 ist hierbei als Akkumulatoreinheit ausgebildet. Hierbei ist die Energiespeichereinheit 42 in der Sohle 54 des Arbeitsschuhs 10 angeordnet (Figur 3). Hierbei kann die Energiespeichereinheit 42 austauschbar in der Sohle 54 des Arbeitsschuhs 10 angeordnet sein. Zudem ist es denkbar, dass die Energiespeichereinheit 42 als externe Energiespeichereinheit ausgebildet ist, die mittels einer Energieversorgungsleitung mit dem Arbeitsschuh 10 verbindbar ist und beispielsweise an einem von einem Schuhträger getragenen Gürtel anordenbar ist. Die Energieversorgungseinheit 40 und/oder die Energiespeichereinheit 42 umfassen zumindest eine Backupeinheit, die dazu vorgesehen ist, Grundfunktionen der aktiven Schutzeinheit 14 in einem Notbetrieb zu ermöglichen, wie beispielsweise eine Ausgabe einer Meldung mittels der Ausgabeeinheit 44 bei einem niedrigen Energieinhalt der Energiespeichereinheit 42 usw.

Der Arbeitsschuh 10 umfasst zumindest die Ausgabeeinheit 44 zu einer Ausgabe von Informationen zumindest in Abhängigkeit von der zumindest einen erfassten personenbezogenen Kenngröße und/oder von der zumindest einen erfassten Umgebungskenngröße und/oder in Abhängigkeit von mittels einer Kommunikationseinheit 30 übertragenen Informationen. Somit sind beispielsweise mittels der Ausgabeeinheit 44 unterschiedliche Temperaturkenngrößen anzeigbar, wobei die Ausgabeeinheit 44 hierzu zumindest ein als Display ausgebildetes Ausgabeelement und/oder ein als Leuchtelement, insbesondere ein als LED, ausgebildetes Ausgabeelement umfasst. Beispielsweise ist eine Bodentemperatur in farblichen Abstufungen anzeigbar, wobei insbesondere blau für eine Temperatur von weniger als 50° C, grün für eine Temperatur von 20° C und rot für eine Temperatur von mehr als 40° C steht. Mittels der Ausgabeeinheit 44 kann somit vorteilhaft eine Warnmeldung bei drohender Gefahr (Untergrund unter Spannung, ätzende Flüssigkeit auf Untergrund, Untergrundtemperatur zu hoch, Glatteisgefahr o. dgl.) ausgegeben werden. Zudem ist mittels der Ausgabeeinheit 44 einem Schuhträger eine optische Rückmeldung gebbar, ob die Kommunikationseinheit 30 mit einer externen Einheit 32 verbunden ist. Somit kann ein Schuhträger vorteilhaft feststellen, ob eine Kommunikationsverbindung zuverlässig zustande gekommen ist. Zusätzlich ist hierbei denkbar, dass die Ausgabeeinheit 44 einem Schuhträger zusätzlich anzeigt, mit welcher Art von externer Einheit die Kommunikationseinheit 30 verbunden ist und kommuniziert.

Ferner umfasst der Arbeitsschuh 10 zumindest eine Beleuchtungseinheit 46 zu einer Ausleuchtung eines Arbeitsbereichs. Die Beleuchtungseinheit 46 umfasst zumindest ein Beleuchtungselement 72, das an einem Zehenbereich des Arbeitsschuhs 10 angeordnet ist und zu einer Ausleuchtung eines Arbeitsbereichs vorgesehen ist. Das Beleuchtungselement 72 ist als LED ausgebildet. Die Beleuchtungseinheit 46 kann eine von einem einzelnen Beleuchtungselement 72 abweichende Anzahl an Beleuchtungselementen 72 aufweisen, die am Arbeitsschuh 10 angeordnet sind und die dazu vorgesehen sind, einen Arbeitsbereich auszuleuchten. Hierbei ist es denkbar, dass die Beleuchtungseinheit 46 manuell von einem Schuhträger steuerbar ist oder dass die Beleuchtungseinheit 46 mittels der Steuer- und/oder Regeleinheit 34 steuer- und/oder regelbar ist, insbesondere zumindest in Abhängigkeit zumindest einer mittels eines Lichtsensorelements (hier nicht näher dargestellt) der Sensoreinheit 16 erfassten Lichtkenngröße.

Zudem umfasst der Arbeitsschuh 10 zumindest eine Projektionseinheit 48, die dazu vorgesehen ist, zumindest eine Information auf einen Untergrund zu projizieren. Mittels der Projektionseinheit 48 sind vorteilhaft im Umfeld eines Schuhträgers befindliche Personen vorteilhaft über eine Gefahr informierbar oder über einen Zustand eines Schuhträgers in einer Notsituation informierbar. Die Projektionseinheit 48 ist vorzugsweise als Beamereinheit ausgebildet. Hierbei kann die Projektionseinheit 48 als Laserbeamereinheit oder als eine andere, einem Fachmann als sinnvoll erscheinende Projektionseinheit ausgebildet sein.

## Patentansprüche

1. Arbeitsschuh, insbesondere Sicherheitsschuh, mit zumindest einer passiven Schutzeinheit (12), die dazu vorgesehen ist, einen Schuhträger passiv zumindest vor mechanischen und/oder elektrischen Belastungen zu schützen, und mit zumindest einer aktiven Schutzeinheit (14), die zumindest eine Sensoreinheit (16) aufweist, die dazu vorgesehen ist, zumindest eine Kenngröße zumindest zu einer Ermöglichung einer Schutzfunktion und/oder einer Komfortfunktion zu erfassen, **dadurch gekennzeichnet, dass** die Sensoreinheit (16) zumindest zu einer Erfassung von zumindest einer personenbezogenen Kenngröße und/oder von zumindest einer Umgebungskenngröße vorgesehen ist.

2. Arbeitsschuh nach Anspruch 1, **dadurch gekennzeichnet, dass** die Sensoreinheit (16) zumindest ein Beschleunigungssensorelement (74) umfasst, das dazu vorgesehen ist, zumindest eine Beschleunigungskenngröße zu erfassen, die zumindest zu einer Auswertung eines sicheren Stands eines Schuhträgers heranziehbar ist.

3. Arbeitsschuh nach Anspruch 1 oder 2, **dadurch gekennzeichnet, dass** die Sensoreinheit (16) zumindest ein Temperatursensorelement (18, 20, 22) umfasst, das dazu vorgesehen ist, zumindest eine Temperaturkenngröße, insbesondere eine Körpertemperatur, eine Untergrundtemperatur und/oder eine Umgebungstemperatur, zu erfassen.

4. Arbeitsschuh nach einem der vorhergehenden Ansprüche, **dadurch gekennzeichnet, dass** die Sensoreinheit (16) zumindest ein Drucksensorelement (24) umfasst, das dazu vorgesehen ist, zumindest eine Drucckenngröße, insbesondere eine von einem Schuhträger auf eine Sohle (54) wirkende Sohlendruckkenngröße, zu erfassen.

5. Arbeitsschuh nach zumindest Anspruch 2 oder 4, **gekennzeichnet durch** zumindest eine Auswerteeinheit (26), die zumindest dazu vorgesehen ist, die erfasste Beschleunigungskenngröße zu einer Erkennung eines sicheren Stand eines Schuhträgers auszuwerten und/oder die erfasste Drucckenngröße zu einer Erkennung einer Sohlenbelastungsverteilung auszuwerten.

6. Arbeitsschuh nach einem der vorhergehenden Ansprüche, **dadurch gekennzeichnet, dass** die Sensoreinheit (16) zumindest ein Ortungssensorelement (28) aufweist, das dazu vorgesehen ist, zumindest eine Positionskenngröße, insbesondere zumindest eine globale Positionskenngröße und/oder zumindest eine relative Arbeitsbereichspositionskenngröße, zu erfassen.

7. Arbeitsschuh nach einem der vorhergehenden Ansprüche, **gekennzeichnet durch** zumindest eine Kommunikationseinheit (30), die zu einer Kommunikation mit zumindest einer externen Einheit (32) zu einem Austausch von elektronischen Daten vorgesehen ist.

8. Arbeitsschuh nach einem der vorhergehenden Ansprüche, **gekennzeichnet durch** zumindest eine Steuer- und/oder Regeleinheit (34), die dazu vorgesehen ist, zumindest einen Arbeitsschuhparameter, insbesondere einen Arbeitsschuhkomfortparameter und/oder einen Arbeitsschuhsicherheitsparameter, in Abhängigkeit von der zumindest einen erfassten personenbezogenen Kenngröße und/oder von der zumindest einen erfassten Umgebungskenngröße anzupassen.

9. Arbeitsschuh nach Anspruch 7 und 8, **dadurch gekennzeichnet, dass** die Steuer- und/oder Regeleinheit (34) dazu vorgesehen ist, mittels der Kommunikationseinheit (30) auf eine zentrale Datenbank zuzugreifen, in der zumindest eine Sicherheits- und/oder Betriebsbereichsregel hinterlegt sind/ist, die zumindest zu einer Anpassung zumindest eines Arbeitsschuhparameters und/oder zu einer Ausgabe von Sicherheitsinformationen mittels einer Ausgabeeinheit (44) verwertbar sind/ist.

10. Arbeitsschuh nach einem der vorhergehenden Ansprüche, **dadurch gekennzeichnet, dass** die Sensoreinheit (16) zumindest teilweise in oder an einem passiven Schutzelement (36, 38) der passiven Schutzeinheit (12) angeordnet ist.

11. Arbeitsschuh nach einem der vorhergehenden Ansprüche, **gekennzeichnet durch** zumindest eine Energieversorgungseinheit (40) und/oder eine Energiespeichereinheit (42), die zumindest zu einer Energieversorgung der aktiven Schutzeinheit (14) vorgesehen sind/ist.

12. Arbeitsschuh nach einem der vorhergehenden Ansprüche, **gekennzeichnet durch** zumindest eine Ausgabeeinheit (44) zu einer Ausgabe von Informationen zumindest in Abhängigkeit von der zumindest einen erfassten personenbezogenen Kenngröße und/oder von der zumindest einen erfassten Umgebungskenngröße und/oder in Abhängigkeit von mittels einer Kommunikationseinheit (30) übertragenen Informationen.

13. Arbeitsschuh nach einem der vorhergehenden Ansprüche, **gekennzeichnet, durch** zumindest eine Beleuchtungseinheit (46) zu einer Ausleuchtung eines Arbeitsbereichs.

14. Arbeitsschuh nach einem der vorhergehenden Ansprüche, **gekennzeichnet durch** zumindest eine Projektionseinheit (48), die dazu vorgesehen ist, zumindest eine Information auf einen Untergrund zu projizieren.

15. Sicherheitssystem mit zumindest einem Arbeitsschuh nach einem der vorhergehenden Ansprüche und mit zumindest einer externen Einheit (32), mit der der Arbeitsschuh zu einem Austausch von elektronischen Daten zumindest mittels zumindest einer Kommunikationseinheit (30) des Arbeitsschuhs kommuniziert.

16. Sicherheitssystem nach Anspruch 15, **dadurch gekennzeichnet, dass** die zumindest eine externe Einheit (32) als tragbare Werkzeugmaschine ausgebildet ist.
